# FASCICULE DE BREVET EUROPEEN

(11) **EP 0 687 184 B1**
(45) Date de publication et mention de la délivrance du brevet: **24.07.2002**
(21) Numéro de dépôt: 94908383.6
(22) Date de dépôt: 28.02.1994
(51) Int. Cl.: A61K 48/00, C12N 15/86, C12N 7/01, A61P 27/02

(54) **ADENOVIRUS RECOMBINANTS ET LEUR UTILISATION EN THERAPIE GENIQUE POUR LE TRAITEMENT DES PATHOLOGIES OCULAIRES**
REKOMBINANTE ADENOVIREN UND DEREN VERWENDUNG IN DER GENTHERAPIE ZUR BEHANDLUNG VON AUGENERKRANKUNGEN
RECOMBINANT ADENOVIRUSES AND USE THEREOF IN GENE THERAPY FOR TREATING EYE DISEASES

(30) Priorité: 03.03.1993 FR 9302438
(43) Date de publication de la demande: 20.12.1995
(73) Titulaire: Aventis Pharma S.A., 92160 Antony (FR); INSTITUT NATIONAL DE LA SANTE ET DE LA RECHERCHE MEDICALE (INSERM), 75654 Paris Cédex 13 (FR)
(72) Inventeur: BRIAND, Pascale, F-75015 Paris (FR); PERRICAUDET, Michel, F-28320 Ecrosnes (FR)
(74) Mandataire: Becker, Philippe
(86) Numéro de dépôt international: FR9400220
(87) Numéro de publication internationale: WO9420146

(56) Documents cités:
- WO-A-90/02551
- WO-A-92/17211
- THE NEW BIOLOGIST vol. 3, no. 3 , Mars 1991 pages 203 - 218 X. BREAKEFIELD AND N. DELUCA 'Herpes simplex virus for gene delivery to neurons'
- EXPERIMENTAL EYE RESEARCH vol. 50, no. 5 , Mai 1990 pages 521 - 532 L. STRAMM ET AL 'beta-Glucuronidase mediated pathway essential for retinal pigment epithelial degradation of glycosaminoglucans'
- SCIENCE. vol. 259 , 12 Février 1993 , LANCASTER, PA US pages 988 - 990 G. LE GAL LA SALLE ET AL 'An adenovirus vector for gene transfer into neurons and glia in the brain'
- BONE MARROW TRANSPLANTATION vol. 9, no. SUP1 , 1992 pages 151 - 152 L. STRATFORD-PERRICAUDET ET AL 'Feasibility of adenovirus-mediated gene transfer in vivo'
- HUMAN GENE TRANSFER vol. 219 , 1991 pages 51 - 61 L. STRATFORD-PERRICAUDET AND M. PERRICAUDET 'Gene transfer into animals: the promise of adenovirus'
- INVESTIGATIVE OPHTHALMOLOGY & VISUAL SCIENCE vol. 34, no. 3 , 9 Mars 1993 pages 473 - 476 D. BOK 'Retinal transplantation and gene therapy'

## Description

La présente invention concerne de virus recombinants, leur préparation et leur utilisation en thérapie génique, pour le transfert et l'expression de gènes au niveau de l'oeil. Elle concerne également des compositions pharmaceutiques comprenant lesdits virus recombinants. Plus particulièrement, la présente invention concerne des virus recombinants défectifs et leur utilisation pour la préparation d'une composition pharmaceutique déstinée au traitement de pathologies oculaires tel que défini dans les revendications.

Le traitement des pathologies oculaires, et notamment des maladies héréditaires constitue un problème non résolu actuellement. Parmi ces pathologies, on peut citer par exemple les rétinites pigmentaires, qui résultent d'altérations génétiques, et pour lesquelles aucun traitement n'est actuellement disponible. Par ailleurs, les pathologies non héréditaires telles que les atteintes post-inflammatoires (dégénérescence rétinienne, etc) ne disposent pas non plus aujourd'hui de traitement adapté. En particulier, si l'on tente d'agir préventivement, notamment au moyen de corticoïdes, on ne dispose actuellement d'aucun moyen satisfaisant pour traiter ces atteintes.

Il est donc important de pouvoir disposer d'outils permettant un traitement spécifique, efficace et localisé des pathologies oculaires. La présente invention apporte une solution avantageuse à ce problème, en démontrant la possibilité de traiter les pathologies oculaires par la thérapie génique.

La thérapie génique consiste à corriger une déficience ou une anormalité (mutation, expression aberrante, etc) par introduction d'une information génétique dans la cellule ou l'organe affecté. Cette information génétique peut être introduite soit in vitro dans une cellule extraite de l'organe, la cellule modifiée étant alors réintroduite dans l'organisme, soit directement in vivo dans le tissu approprié. Dans ce second cas, différentes techniques existent, parmi lesquelles des techniques diverses de transfection impliquant des complexes d'ADN et de DEAE-dextran (Pagano et al., J.Virol. 1 (1967) 891), d'ADN et de protéines nucléaires (Kaneda et al., Science 243 (1989) 375), d'ADN et de lipides (Felgner et al., PNAS 84 (1987) 7413), l'emploi de liposomes (Fraley et al., J.Biol.Chem. 255 (1980) 10431), etc. Plus récemment, l'emploi de virus comme vecteurs pour le transfert de gènes est apparu comme une alternative prometteuse à ces techniques physiques de transfection. A cet égard, différents virus ont été testés pour leur capacité à infecter certaines populations cellulaires. En particulier, les rétrovirus (RSV, HMS, MMS, etc), le virus HSV, les virus adéno-associés, et les adénovirus.

Toutefois, jusqu'à présent, aucun de ces vecteurs n'a été utilisé ni décrit comme utilisable pour le transfert de gènes au niveau de l'oeil. La présente invention constitue la première démonstration qu'il est possible de traiter les pathologies oculaires par la thérapie génique.

Un premier objet de l'invention réside dans l'utilisation d'un adénovirus recombinant défectif contenant un gène inséré pour la préparation d'une composition pharmaceutique destinée au traitement des pathologies oculaires, la composition étant adaptée pour administrer ledit adénovirus dans l'oeil.

Plus particulièrement, on utilise selon la présente invention des virus recombinants défectifs dérivés de virus capables d'infecter et d'exprimer un gène inséré dans les cellules de l'oeil, sans entraîner des phénomènes cytopathologiques ou d'effets pathogènes. Des virus susceptibles d'être utilisés dans l'invention sont les adénovirus.

La présente invention repose plus particulièrement sur la mise en évidence que les virus de type adénovirus sont capables de transférer et d'exprimer des gènes désirés au niveau de l'oeil. Les exemples présentés plus loin montrent que les adénovirus sont capables, selon le mode d'administration, de transférer efficacement, pour une durée importante et sans effet cytopathologique, des gènes dans l'endothélium cornéen, dans les cellules photoréceptrices, dans les cellules du nerf optique, dans les cellules bipolaires, etc. Par ailleurs, compte tenu de l'accès relativemant aisé aux différents compartiments de l'oeil par la microchirurgie (microinjection), ainsi que de l'existence de barrières naturelles dans cet organe (membrane de Descemet, membrane de Bruch, cristallin, etc) la présente invention permet avantageusement d'effectuer un transfert de gènes très ciblé, en fonction de la pathologie à traiter. Les résultats présentés montrent également que l'expression d'un gène désiré est stable sur une longue période (aucune perte d'activité à 50 jours).

Dans un mode de réalisation préféré, l'invention réside dans l'utilisation d'un adénovirus recombinant défectif contenant un gène inséré pour la préparation d'une composition pharmaceutique destinée au traitement des pathologies oculaires tel que défini dans les revendications.

Le terme "virus ou adénovirus défectif" désigne un virus incapable de se répliquer de façon autonome dans la cellule cible. Généralement, le génome des virus défectifs utilisés dans le cadre de la présente invention est donc dépourvu au moins des séquences nécessaires à la réplication dudit virus dans la cellule infectée. Ces régions peuvent être soit éliminées (en tout ou en partie), soit rendues non-fonctionnelles, soit substituées par d'autres séquences et notamment par le gène inséré. Préférentiellement, le virus défectif conserve néanmoins les séquences de son génome qui sont nécessaires à l'encapsidation des particules virales.

S'agissant des adénovirus, il en existe différents sérotypes, dont la structure et les propriétés varient quelque peu. Néanmoins, ces virus ne sont pas pathogènes pour l'homme, et notamment les sujets non immuno-déprimés. Parmi ces sérotypes, on préfère utiliser dans le cadre de la présente invention les adénovirus de type 2 ou 5 (Ad 2 ou Ad 5). Dans le cas des adénovirus Ad 5, les séquences nécessaires à la réplication sont les régions E1A et E1B.

Des virus recombinants défectifs dérivés de rétrovirus, de virus adéno-associés ou du virus HSV (herpes simplex virus) ont déjà été décrits dans la littérature [Roemer et Friedmann, Eur. J. Biochem. 208 (1992) 211 ; Dobson et al., Neuron 5 (1990) 353 ; Chiocca et al., New Biol. 2 (1990) 739 ; Miyanohara et al., New Biol. 4 (1992) 238; WO91/18088].

Au sens de la présente invention, le terme "gène inséré" désigne toute séquence d'ADN introduite dans le virus recombinant, dont l'expression dans la cellule cible est recherchée.

Il peut s'agir en particulier d'un (ou plusieurs) gène(s) de structure codant pour une (des) protéine(s) ou pour une partie d'une (de) protéine(s). La protéine ou partie de protéine ainsi codée peut être une protéine homologue vis-à-vis de la cellule cible (c'est-à-dire une protéine qui est normalement exprimée dans la cellule cible lorsque celle-ci ne présente aucune pathologie), ou une protéine hétérologue vis-à-vis de ladite cellule. Dans le premier cas, l'expression de la protéine permet par exemple de pallier une expression insuffisante dans la cellule ou l'expression d'une protéine inactive ou faiblement active en raison d'une modification, ou encore de surexprimer ladite protéine. Dans le second cas, la protéine exprimée peut par exemple compléter ou apporter une activité déficiente dans la cellule lui permettant de lutter contre une pathologie.

Parmi les gènes insérés au sens de la présente invention, on peut citer plus particulièrement :
- les gènes impliqués dans des pathologies génétiques oculaires,
- les gènes codant pour des facteurs de croissance, des cytokines ou des neurotrophines : Le role protecteur ou curatif du produit d'expression de ces gènes dans différentes pathologies oculaires a été démontré, et notamment sur la détérioration des cellules photoréceptrices sous l'effet de la lumière (Lavail et al., PNAS 89 (1992) 11249).
- les gènes de facteurs de régulation (facteurs de transcription, facteurs de traduction),
- les gènes codant pour des enzymes,
- les gènes codant pour des protéines ayant des propriétés anticancéreuses, telles que les interférons, les facteurs de nécrose des tumeurs, etc, ou encore,
- les gènes codant pour des antigènes permettant une vaccination (une protection) locale contre une infection de l'oeil.

A titre d'exemples spécifiques, mais non limitatifs, on peut citer :
. le gène de l'ornithine aminotransférase impliqué dans l'atrophie gyrée (Akaki et al., J. Biol. Chem. 267(18) (1992) 12950),
. le gène de la rhodopsine, impliqué dans une forme de rétinite pigmentaire (Dryja et al., Nature 343 (1990) 364),
. le gène de la périphérine RDS, impliqué dans une forme de rétinite pigmentaire (Farrar et al., Nature 354 (1991) 478),
. le gène de la tyrosinase, impliqué dans l'albinisme oculocutanée type B1 (Giebel et al., Am. J. Hum. Genet. 48 (1991) 1159),
. le gène mitochondrial NDI, impliqué dans la maladie de Leber (Howell et al., Am. J. Hum. Genet. 48 (1991) 935),
. le gène de la sous-unité β de la cGMP phosphodiestérase, qui permet de ralentir la dégénérescence rétinienne (Lem et al., PNAS 89 (1992) 4422),
. le gène de la rab géranylgéranyl transférase, dont la déficience semble liée à une dégénérescence rétinienne lors de choroïdermies (Seabra et al., Science 259 (1993) 377),
. le gène du facteur de croissance des fibroblastes basique (bFGF), capable de retarder la dégénérescence des cellules photoréceptrices observée dans certaines dystrophies rétiniennes héréditaires (Faktorovich et al., Nature 347 (1990) 83),
. le gène de l'interleukine-8, qui permet d'induire une néovascularisation dans la cornée (Strieter et al., Am. J. Pathol. 141(6) (1992) 1279).

Le terme "gène inséré" désigne également des séquences antisens, dont l'expression dans la cellule cible permet de contrôler l'expression de gènes ou la transcription d'ARNm cellulaires. De telles séquences peuvent par exemple être transcrites, dans la cellule cible, en ARN complémentaires d'ARNm cellulaires et bloquer ainsi leur traduction en protéine.

Généralement, le gène inséré comprend également des séquences permettant son expression dans la cellule infectée. Il peut s'agir des séquences qui sont naturellement responsables de l'expression dudit gène lorsque ces séquences sont susceptibles de fonctionner dans la cellule infectée. Il peut également s'agir de. séquences d'origine différente (responsables de l'expression d'autres protéines, ou même synthétiques). Notamment, il peut s'agir de séquences issues du génome de la cellule que l'on désire infecter, ou du génome du virus utilisé. S'agissant d'adénovirus, on peut citer par exemple les promoteurs des gènes E1A, MLP, etc. En outre, ces séquences d'expression peuvent être modifiées par addition de séquences d'activation, de régulation, etc. Par ailleurs, lorsque le gène inséré ne comporte pas de séquences d'expression, il peut être inséré dans le génome du virus défectif en aval d'une telle séquence.

Dans ce qui suit sont décrites plus en détail la construction et l'utilisation d'adénovirus recombinants défectifs. Il est entendu néanmoins que cette description peut être appliquée par l'homme du métier aux autres virus suceptibles d'être utilisés dans le cadre de la présente invention, ainsi qu'indiqués plus haut.

Les adénovirus recombinants défectifs peuvent être préparés par recombinaison homologue entre un adénovirus et un plasmide portant entre autre le gène que l'on désire insérer. La recombinaison homologue se produit après co-transfection desdits adénovirus et plasmide dans une lignée cellulaire appropriée. La lignée cellulaire utilisée doit de préférence (i) être transformable par lesdits éléments, et (ii), comporter les séquences capables de complémenter la partie du génome de l'adénovirus défectif, de préférence sous forme intégrée pour éviter les risques de recombinaison. A titre d'exemple de lignée, on peut mentionner la lignée de rein embryonnaire humain 293 (Graham et al., J. Gen. Virol. 36 (1977) 59) qui contient notamment, intégrée dans son génome, la partie gauche du génome d'un adénovirus Ad5 (12 %).

Ensuite, les vecteurs qui se sont multipliés sont récupérés et purifiés selon les techniques classiques de biologie moléculaire.

La présente invention concerne également une composition pharmaceutique comprenant une quantité suffisante de virus recombinant défectif tel que décrit précédemment, sous une forme adaptée à un usage oculaire. par administration dans l'oeil.

En particulier, le virus recombinant défectif peut être sous forme de solution injectable, de collyre, de pommade ophtalmique, etc. Les véhicules pharmaceutiquement acceptables pour de telles formulations adaptées à un usage oculaire sont notamment des solutions salines (phosphate monosodique, disodique, clrorure de sodium, potassium, calcium ou magnésium, etc, ou des mélanges de tels sels), la vaseline, l'huile de vaseline, etc.

Dans le cas de collyres ou de pommades ophtalmiques, il est entendu que les applications thérapeutiques peuvent être plus limitées en raison d'une diffusion plus faible du virus recombinant défectif.

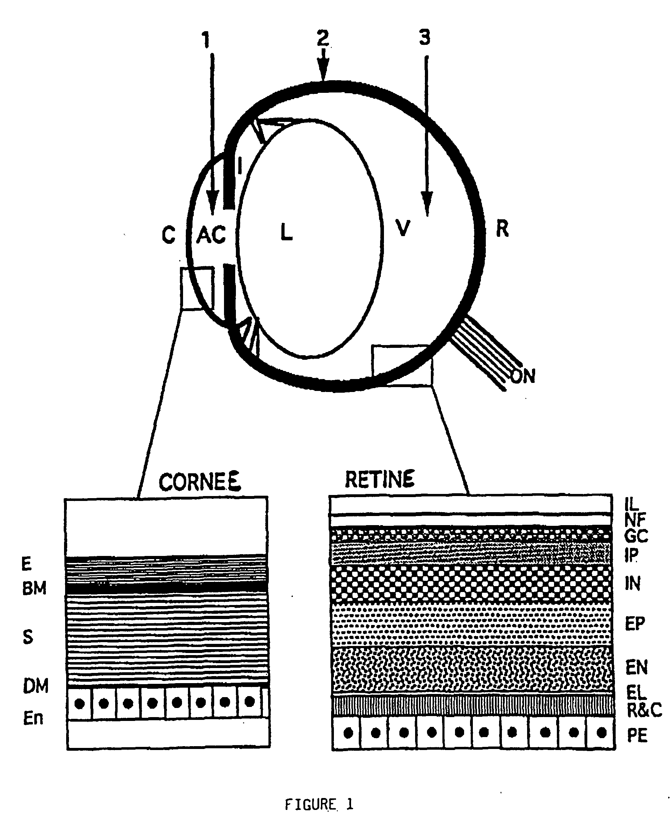

Dans leur utilisation pour le traitement des pathologies oculaires, les virus recombinants défectifs selon l'invention peuvent être administrés selon différents modes, et notamment par injection sousrétinale, éventuellement précédée d'une vitrectomie, ou par injection intravitreuse, simples ou multiples (voir figure 1). L'injection sous-rétinale peut être réalisée sélectivement dans différents compartiments de l'oeil, et notamment, l'injection peut être réalisée au niveau du vitré, de la chambre anterieure ou de l'espace rétrobulbaire. Les résultats présentés dans la présente demande montrent que ces différents modes d'injection permettent d'infecter de manière ciblée les différents tissus de l'oeil, et notamment, l'endothélium cornéen, les cellules photoréceptrices, les cellules bipolaires, les cellules ganglionaires ou encore les cellules des muscles oculomoteurs.

Les doses de virus utilisées pour l'injection peuvent être adaptées en fonction de différents paramètres, et notamment en fonction du mode d'administration utilisé, de la pathologie concernée, du gène à exprimer, ou encore de la durée du traitement recherchée. D'une manière générale, les adénovirus recombinants selon l'invention sont formulés et administrés sous forme de doses comprises entre 10⁴ et 10¹⁴ pfu/ml, et de préférence 10⁶ à 10¹⁰ pfu/ml. Le terme pfu ("plaque forming unit") correspond au pouvoir infectieux d'une solution de virus, et est déterminé par infection d'une culture cellulaire appropriée, et mesure, généralement après 48 heures, du nombre de plages de cellules infectées. Les techniques de détermination du titre pfu d'une solution virale sont bien documentées dans la littérature.

Compte tenu de la stabilité d'expression du gène inséré dans la cellule cible, la présente invention devrait permettre de traiter la majorité des pathologies oculaires avec peu d'injections.

La présente invention offre ainsi un moyen très efficace pour le traitement des pathologies oculaires, et notamment celles dont les mécanismes ont été élucidés au niveau moléculaire. En particulier, l'implication de gènes a été démontrée dans l'atrophie gyrée, dans la maladie de Norrie (Hum. Mol. Genet. 1(7) (1992) 461), dans la dégénérescence rétinienne (Bowes et al., PNAS 86 (1989) 9722), dans la maladie de Leber, dans les choroïdermies (Cremers et al., Nature 347 (1990) 674), dans la dégénérescence des cellules photoréceptrices, dans les rétinites pigmentaires, dans l'albinisme, dans le syndrome Kearns-Sayre (Shoffner et al., PNAS 86 (1989) 7952), etc. La présente invention est également pour le traitement des altérations de la cornée acquises résultant de maladies inflammatoires, des atteintes rétiniennes post-inflammatoires, etc.

La présente invention rend également possible la thérapie par les protéines ou peptides, dont l'utilisation par les voies classiques d'administration est très hypothétique en raison de leur forte sensibilité aux mécanismes de dégradation et d'élimination de l'organisme, et des problèmes liés à la pénétration dans les cellules. L'emploi de virus selon l'invention permet d'exprimer directement à l'interieur de la population de cellules ciblées le polypeptide ou la protéine désirée, qui n'est donc plus accessible aux mécanismes mentionnés ci-avant.

L'ensemble des résultats présentés dans la présente demande démontre plus particulièrement que les adénovirus recombinants, défectifs pour la réplication, constituent des vecteurs particulièrement intéressants pour le transfert de gènes in vivo dans les cellules oculaires. Les expériences réalisées montrent la possibilité d'une expression stable à long-terme de gènes dans ces cellules. En particulier, une expression stable est observée 50 jours après l'injection. De plus, le spectre d'expression large dans les différentes cellules oculaires constitue également un résultat particulièrement intéréssant dans la mesure où pratiquement toutes les maladies de la rétine (notamment la retinitis pigmentosa) affectent une grande surface de la rétine.

En outre, ce traitement peut concerner aussi bien l'homme que tout animal tel que les ovins, les bovins, les animaux domestiques (chiens, chats, etc), les chevaux, les poissons, etc.

La présente invention est plus complètement décrite à l'aide des exemples qui suivent, qui doivent être considérés comme illustratifs et non limitatifs.

### Légende des figures

Figure 1 : Représentation schématique de l'oeil. C = cornée; AC = Chambre antérieure; L = cristallin; V = vitré; I = iris; ON = nerf optique; R = espace rétrobulbaire.

### Construction d'un adénovirus recombinant défectif (Ad,RSVβGal) :

La procédure générale permettant la préparation des adénovirus recombinants a été décrite dans la partie générale de la description.

L'adénovirus Ad.RSVβGal est un adénovirus recombinant défectif (délété des régions E1 et E3 ) obtenu par recombinaison homologue in vivo entre l'adénovirus mutant Ad-d1324 (Thimmappaya et al., Cell 31 (1982) 543) et le plasmide pAd.RSVβGal (Akli et al. 1993).
Le plasmide pAd.RSVβGal contient, dans l'orientation 5'->3',
- le fragment PvuII correspondant à l'extrémité gauche de l'adénovirus Ad5 comprenant : la séquence ITR, l'origine de réplication, les signaux d'encapsidation et l'amplificateur E1A;
- le gène codant pour la β-galactosidase sous le contrôle du promoteur RSV (du virus du sarcome de Rous),
- un second fragment du génome de l'adénovirus Ad5, qui permet la recombinaison homologue entre le plasmide pAd.RSVβGal et l'adénovirus d1324.

Après linéarisation par l'enzyme ClaI, le plasmide pAd.RSVβGal et l'adénovirus d1324 sont co-transfectés dans la lignée 293 en présence de phosphate de calcium pour permettre la recombinaison homologue. Les adénovirus recombinants ainsi générés sont sélectionnés par purification sur plaque. Après isolement, l'ADN de l'adénovirus recombinant est amplifié dans la lignée cellulaire 293, ce qui conduit à un surnageant de culture contenant l'adénovirus défectif recombinant non purifié ayant un titre d'environ 10¹⁰ pfu/ml.

Les particules virales sont généralement purifiées par centrifugation sur gradient de chlorure de césium selon les techniques connues (voir notamment Graham et al., Virology 52 (1973) 456). L'adénovirus Ad.RSVβGal est conservé à -80°C dans 20 % de glycérol. Avant injection, la suspension d'adénovirus est diluée au tiers dans un tampon phosphate PBS.

### Injection in vivo

### - Protocole

Des souris C57Bl/6 de 3 à 7 semaines ont été anesthésiées avec de l'avertine. Dans chaque oeil a ensuite été injecté 10⁷ à 10⁸ pfu d'adénovirus recombinant Ad.RSVβGal, soit au niveau de la chambre anterieure, soit au niveau du vitré, soit au niveau de l'espace rétrobulbaire (voir figure 1). Les animaux ont été sacrifiés 7 à 50 jours après l'injection par dislocation cervicale et les yeux ont été récupérés et fixés dans l'azote liquide. Des sections sagitales et coronales (10-15 µm) sont réalisées sur cryostat, puis colorées en présence de X-gal pour révéler l'activité β-galactosidase qui peut être visualisée par l'apparition d'une coloration bleue dans le noyau des cellules infectées, et contre-colorées avec de l'hémotoxyline et de l'éosine.

### - Injection au niveau de la chambre anterieure

Après injection de 10⁸ pfu d'adénovirus Ad.RSVβGal au niveau de l'espace de la chambre anterieure, seules les cellules de la couche endotheliale expriment l'activité β-galactosidase. En revanche, les cellules épithéliales ou du stroma ne présentent aucune coloration à la suite d'une telle injection. De plus, les cellules marquées (infectées) sont distribuées régulièrement dans la couche endothéliale, quel que soit le temps d'administration. Ce résultat montre que la présente invention permet de transférer et d'exprimer un gène dans les cellules endothéliales de l'oeil.

### - Injections intravitreuses

Des injections intravitreuses ont également été réalisées, dans le but d'infecter différents types de cellules de la rétine. Contrairement à la distribution uniforme dans les cellules endothéliales après injection au niveau de l'espace chambre anterieure, la distribution des cellules positives (infectées) après injection intravitreuse est limitée à l'hémirétine correspondant au point d'injection. La taille importante du cristallin et les caractéristiques de viscosité de l'humeur vitrée pourraient expliquer cette expression confinée. Cependant, lorsque des injections temporales et nasales sont effectuées simultanément, les cellules des 2 hémirétines sont infectées. Ces résultats montrent donc qu'il est possible de transférer et d'exprimer un gène au niveau de la rétine. Ils montrent également que, selon la pathologie à traiter, et notamment selon sa distribution sur la rétine, il est possible de cibler le transfert sur une hémirétine seulement.

Trois couches nucléaires, correspondant aux cellules ganglionaires, bipolaires et photoréceptrices présentent également une coloration intense à trois semaines (age à partir duquel le développement de la rétine est terminé), ainsi que chez les souris adulte. Malgré la présence du signal permettant la localisation nucléaire de la protéine LacZ, le marquage (et donc l'infection) de certaines cellules au niveau du site d'injection est si intense que la coloration diffuse dans le cytoplasme. Pour cette raison, le couche de fibre nerveuse correspondant aux axones des noyaux marqués (qui convergent pour former le nerf optique) est marquée de manière homogène.

Une analyse fine des différentes couches de cellules rétiniennes ne fait apparaitre aucune diminution significative de leur épaisseur. De plus, la tête du nerf optique n'est pas altérée, même à des doses élevées d'adénovirus (10⁷ pfu).

### - Injection au niveau de l'espace rétrobulbaire

Pour évaluer la possibilité d'une diffusion du virus à travers la sclera, des souris ont été injectées au niveau de l'espace rétrobulbaire. Contrairement à la coloration rétinienne, environ 100 % des fibres des 4 muscles oculomoteurs ont été infectées et expriment l'activité β-galactosidase.

L'ensemble de ces résultats démontre clairement que les adénovirus recombinants, défectifs pour la réplication, constituent des vecteurs particulièrement intéressants pour le transfert de gènes in vivo dans les cellules oculaires.

## Revendications

1. Utilisation d'un adénovirus recombinant défectif contenant un gène inséré pour la préparation d'une composition pharmaceutique destinée au traitement des pathologies oculaires et adaptée à l'administration dudit adénovirus dans l'oeil.

2. Utilisation selon la revendication 1 **caractérisée en ce que** l'adénovirus recombinant défectif est dépourvu des régions de son génome qui sont nécessaires à sa réplication dans la cellule infectée.

3. Utilisation selon les revendications 1 ou 2 **caractérisée en ce que** l'adénovirus recombinant défectif est un adénovirus de type Ad 2.

4. Utilisation selon la revendication 1 ou 2 **caractérisée en ce que** l'adénovirus recombinant défectif est un adénovirus de type Ad 5.

5. Utilisation selon l'une des revendications 1 à 4 **caractérisée en ce que** le gène inséré comprend des séquences permettant son expression dans la cellule infectée.

6. Utilisation selon l'une des revendications 1 à 5 **caractérisée en ce que** le gène inséré code pour une protéine ou un fragment de protéine.

7. Utilisation selon l'une des revendications 1 à 5 **caractérisée en ce que** le gène inséré est une séquence antisens.

8. Utilisation selon la revendication 1 pour la préparation d'une composition pharmaceutique destinée au traitement des pathologies héréditaires telles que les rétinites pigmentaires.

9. Utilisation selon l'une des revendications 1 à 8 **caractérisée en ce que** l'adénovirus est adapté à l'administration par injection

10. Utilisation selon l'une des revendications 1 à 8 **caractérisée en ce que** l'adénovirus est adapté à l'administration par injection sous-rétinale ou par injection intravitreuse.

11. Utilisation selon l'une des revendications 1 à 8 **caractérisée en ce que** l'adénovirus est adapté à l'administration dans l'endothélium cornéen, les cellules photoréceptrices, les cellules bipolaires, les cellules ganglionaires ou les cellules des muscles oculomoteurs.

## Patentansprüche

1. Verwendung eines defektiven, rekombinanten Adenovirus, enthaltend ein insertiertes Gen, für die Herstellung einer pharmazeutischen Zusammensetzung zur Behandlung von Augenerkrankungen, die an die Verabreichung des genannten Adenovirus in das Auge angepaßt ist.

2. Verwendung nach Anspruch 1, **dadurch gekennzeichnet, daß** der defektive, rekombinante Adenovirus von Bereichen seines Genoms befreit ist, die für seine Replikation in die infizierte Zelle erforderlich sind.

3. Verwendung nach Anspruch 1 oder 2, **dadurch gekennzeichnet, daß** der defektive, rekombinante Adenovirus ein Adenovirus vom Typ Ad 2 ist.

4. Verwendung nach Anspruch 1 oder 2, **dadurch gekennzeichnet, daß** der defektive, rekombinante Adenovirus ein Adenovirus vom Typ Ad 5 ist.

5. Verwendung nach einem der Ansprüche 1 bis 4, **dadurch gekennzeichnet, daß** das insertierte Gen Sequenzen umfaßt, die seine Expression in die infizierte Zelle gestatten.

6. Verwendung nach einem der Ansprüche 1 bis 5, **dadurch gekennzeichnet, daß** das insertierte Gen für ein Protein oder ein Fragment eines Proteins kodiert.

7. Verwendung nach einem der Ansprüche 1 bis 5, **dadurch gekennzeichnet, daß** das insertierte Gen eine Gegensinn-Sequenz ist.

8. Verwendung nach Anspruch 1 zur Herstellung einer pharmazeutischen Zusammensetzung zur Behandlung von erblich bedingten Erkrankungen wie pigmentärer Retinitis.

9. Verwendung nach einem der Ansprüche 1 bis 8, **dadurch gekennzeichnet, daß** der Adenovirus an die Verabreichung durch Injektion angepaßt ist.

10. Verwendung nach einem der Ansprüche 1 bis 8, **dadurch gekennzeichnet, daß** der Adenovirus an die Verabreichung durch subretinale Injektion oder durch Injektion in den Glaskörper angepaßt ist.

11. Verwendung nach einem der Ansprüche 1 bis 8, **dadurch gekennzeichnet, daß** der Adenovirus an die Verabreichung in das Hornhaut-Endothelium, in die Photorezeptor-Zellen, in die bipolaren Zellen, in die Ganglion-Zellen oder in die Zellen der oculomotorischen Muskeln angepaßt ist.

## Claims

1. Use of a defective recombinant adenovirus containing an inserted gene for the preparation of a pharmaceutical composition intended for the treatment of ocular pathologies and suitable for administration of the said adenovirus into the eye.

2. Use according to Claim 1, **characterized in that** the defective recombinant adenovirus lacks the regions of its genome which are needed for its replication in the infected cell.

3. Use according to Claim 1 or 2, **characterized in that** the defective recombinant adenovirus is a type Ad 2 adenovirus.

4. Use according to Claim 1 or 2, **characterized in that** the defective recombinant adenovirus is a type Ad 5 adenovirus.

5. Use according to one of Claims 1 to 4, **characterized in that** the inserted gene comprises sequences permitting its expression in the infected cell.

6. Use according to one of Claims 1 to 5, **characterized in that** the inserted gene codes for a protein or a protein fragment.

7. Use according to one of Claims 1 to 5, **characterized in that** the inserted gene is an antisense sequence.

8. Use according to Claim 1 for the preparation of a pharmaceutical composition intended for the treatment of hereditary pathologies such as retinitis pigmentosa.

9. Use according to one of Claims 1 to 8, **characterized in that** the adenovirus is suitable for administration by injection.

10. Use according to one of Claims 1 to 8, **characterized in that** the adenovirus is suitable for administration by subretinal injection or by intravitreous injection.

11. Use according to one of Claims 1 to 8, **characterized in that** the adenovirus is suitable for administration into the corneal endothelium, the photoreceptor cells, the bipolar cells, the ganglion cells or the cells of the oculomotor muscles.
